# EUROPEAN PATENT SPECIFICATION

(11) **EP 1 128 860 B1**
(45) Date of publication and mention of the grant of the patent: **23.06.2004**
(21) Application number: 99955844.8
(22) Date of filing: 18.11.1999
(51) Int. Cl.: A61M 5/32

(54) **INJECTION NEEDLE**
INJEKTIONSNADEL
AIGUILLE A INJECTION

(30) Priority: 20.11.1998 DK 152498
(43) Date of publication of application: 05.09.2001
(73) Proprietor: NOVO NORDISK A/S, 2880 Bagsvaerd (DK)
(72) Inventor: LARSEN, Andre, DK-2791 Dragor (DK)
(74) Representative: Hansen, Einar Tronier
(86) International application number: PCT/DK1999/000634
(87) International publication number: WO 2000/030705

(56) References cited:
- WO-A1-97/39787
- DATABASE WPI ACCESSION NO. 1993-163626 FURUKAWA ELECTRIC CO LTD.: 'Syringe assembly for analysing small samples - comprises syringe having fluid injecting needle made of ultra-elastic material contg. a nickel-titanium alloy and plunger', XP002947409 & JP 5 096 004 A 20 April 1993

## Description

The invention relates to injection needle units for use on a syringe by patient administrated injections. Such needle units comprises a hub fitting on a syringe and a needle mounted in the hub with pointed ends projecting from each end of the hub, a first end designed for penetrating the seal of a cartridge in a syringe and a second end designed for insertion into the skin of a patient to inject medicine from said cartridge.

As many people has needle phobia which makes them reluctant to inject themselves it is attempted to develop needles which does not leave a pain experience which can amplify this needle phobia.

Less pain by needle insertion is obtained by making the needles still thinner and shorter. Whereas a lower limit for the length of the needles limit is set by the fact that an injection needle must be long enough to penetrate the skin and to deliver the medication subcutaneously, the limit for the thickness of the needle is set by the fact that a medicine shall be able to flow though the needle within an acceptable time and that the needle shall posses a mechanical strength so that it does not brake or collapse when it is influenced by bending forces during the insertion and injection.

As it appears to be possible to make an insulin suspension and consequently other medicament solutions flow through needles as thin as G30 and even thinner, i.e. needles having outer diameters less than 0.320 mm and an inner diameter preferably larger than 0,13 mm, the mechanical strength sets the crucial limit for the thickness of a useable injection needle. Consequently standards are made which tell how far stainless steel injection needles for medical use are allowed to bend when influenced by defined forces.

It is an object of the invention to provide a needle having an outer diameter thinner than 0.320 mm which will not be in danger of braking even when the limits set by ISO 9626 are exceeded.

This is obtained by an injection needle unit for use on a syringe by patient administrated injections comprising a hub fitting on a syringe which hub carries a needle having a cross section diameter less than 0,320 mm and being mounted in the hub with pointed ends projecting from each end of the hub a first end designed for penetrating the seal of a cartridge in a syringe and a second end designed for insertion into the skin of a patient to inject medicine from said cartridge, wherein the needle according to the invention is made from an super elastic material which can obtain recovered elongation of more than 2% without having permanent deformation in the material. This elastic deformation can also be defined through Hooke's law σ = E x ε, where σ is the strain , E is the Elasticity Modulus (Young's Modulus or Secant Modulus) and ε is the elongation. **σ** and ε will be proportional with constant E-modulus when having a fully elastic deformation phase.

Such a super elastic behaviour will e.g. be beneficial during repetitive bending of the needle to angles exceeding the limits defined in ISO 9626, and needles made from such super elastic material can be bent at rather sharp angles without breaking or collapsing and will after it have been bent find back to its linear shape.

According to the invention the needle may be made from a super elastic material from a special group of materials which in a given temperature interval change their crystal structure.

According to the invention the super elastic material used may be a NiTi alloy.

Use of super elastic needles for syringes is known from Japanese Patentapplication No. 5-96004 which concerns injectors for analytical purpose designated as micro syringes and used to accurately measure small volumes of samples for injection into measuring instruments, some of which are capable of measuring a volume in the order of 0.1 microliters.

The high form stability and stiffness of an NiTi alloy containing 50.2 to 52.0 at. % Ni is taken advantage of in a needle with a diameter of 0.5 mm which maintains its linear shape when the needle is inserted into a needle guide of the injection device on sample injection into the measuring instrument. It is important that the needle maintains its linear shape as the piston can be moved into the lumen of the needle.

In needles for subcutaneous injections it is important that the needle is thin. Needles thinner than 0.320 mm are aimed at whereas it is less important if the needle is bent during the insertion if only it can be ensured that the needle do not break or collapse. This makes it attractive to use an alloy with a higher Ni content as needles made from such an alloy are less stiff but very elastic so that large deformations can be tolerated without any risk for breakage.

According to the invention an alloy containing at least 52 % Ni and consisting of the balance Ti and unavoidable impurities is found appropriate.

The properties, inbreakability and elasticity, of the needle may be further enhanced by adding at least one further element at a content not higher than 4%.

In excess of being practically unbreakable the NiTi needle in spite of its large Ni content has shown to have a better biocompatibility than has ordinary stainless steel for medical purposes. Further the torsion strength and the ultimate strength of a NiTi needle is appreciably higher than for a corresponding needle made from conventional stainless steel.

Alternatively the needle may according to the invention be made from a curable or thermoplastic polymer super elastic material.

The needle hub may be of the kind comprising a sleeve with an internal thread, which sleeve forms the attachment means and can be screwed onto an outer thread on an attachment part of a syringe, and which hub has a basis carrying the sleeve and the needle. The needle is fixed in the basis with an injection part projecting from one side of said basis and a back needle projecting from the other side of the basis the back needle being surrounded by the sleeve which projects from the basis perpendicularly to said basis on the same side as the back needle. When the hub is screwed onto a pen the back needle may penetrate a rubber membrane which closes an ampoule in the pen. A medicine, e. g. Insulin, can now be administered from the ampoule through the needle.

In an embodiment of the needle hub a needle guide may be provided which needle guide has an abutment surface by which it can abut the skin where the injection is going to be made, and a needle guide channel opening through said abutment surface. The needle which is movable relative to the abutment surface can be moved through the needle guide to make a sharpened end of the needle project from said surface. When the surface abuts the skin the needle will project through the skin into the underlying tissue. The needle guide may hide the needle to the user and the abutment of the abutment surface may distract the user so that he hardly feels the sting when the needle is inserted.

A small cavity may be provided in the abutment surface around the opening of the needle guide which may fit to the outer diameter of the needle so that air but hardly liquid can pass between the needle and the guide. The opening may be covered by a transparent cover plate which is frosted on its side facing the cavity. Before insertion of the needle its sharp end is positioned in the cavity beneath the cover plate. When a hub with a needle guide and with the above mentioned cavity covered with a frosted cover plate is mounted on a syringe it may be used as an air shot indicator. Air shots are made to ensure that air is driven out of the ampoule and the needle before attempt are made to inject medicine. To make an air shot a small dose is set and is pressed out through the needle the syringe being held with the injection part of the needle pointing vertically upward. This process is repeated until a thin jet of liquid is seen to leave the sharp end of the needle. When a needle with an air shot indicator of the above mentioned kind is used, air from the needle will pass away through the narrow gap between needle and needle guide. When liquid leaves the sharp end of the needle the cavity will quickly be filled and wet and the frosted surface which will then change appearance to be more clear. When the needle is going to be inserted through the skin it is passed through the cover plate and into the skin.

In another embodiment the cavity in which the sharp end of the needle is positioned and which is covered by a cover plate may contain a lubricant that makes the needle easier penetrate the skin, or the cavity may contain an anaesthetic to make the needle insertion totally painless.

In still another embodiment the elasticity of the needle may be taken advantage of by providing an enforced curved course of the injection part of the needle between the basis of the needle hub and the needle guide. With the enforced curved course the sharp point of the injection part is positioned immediately beneath the abutment surface which is held against the skin. When the needle is going to be inserted the enforcement is released and the needle will due to its elasticity straighten itself an thereby move the sharp end through the skin. If the needle itself does not posses the force needed for the straightening and insertion of the needle the straightening may be supported by a conventional spring element.

To ensure that the super elastic back needle do not deflect when the needle hub is mounted on a syringe where the super elastic needle shall penetrate the rubber membrane sealing a cartridge in the syringe a reinforcement of the back needle may be provided.

The reinforcement of the back needle may comprise a plastic needle integral with the needle hub surrounding the back needle of the super elastic needle injection needle or it may be a conventional stainless steel needle mounted in the needle hub in continuation of the super elastic needle or surrounding the back needle of said needle.

An embodiment of a needle unit may according to the invention comprise a needle hub comprising a basis having a first and a second side, a sleeve projecting from the first side of the basis and forming attachment means by which the needle unit can be mounted onto an attachment part of a syringe, and a housing projecting from the second side of the basis in which housing a plug is axially displaceable, a needle fixed in the basis with a part forming a back needle projecting from the first side of the basis and being surrounded by the sleeve, and rest of the needle projecting from the second side of the basis and being surrounded by the housing, the needle further being fixed in the plug with an injection part projecting from a distal end of the plug and an intermediary part running between a proximal end of the plug and the second side of the basis.

When the plug is moved into the housing, the needle is forced to bend out from its linear shape. If the plug is released the needle will seek to return to its linear form whereby the plug and the injection needle part is moved in a distal direction. When the needle unit is held with its distal end against the skin where an injection is wanted, said movement in the distal direction will make the injection needle penetrate the skin and be ready for an injection of a medicine through the needle.

To avoid sharp bending of the intermediary needle at the transition between this needle and the basis and the plug flared recesses may be provided around the needle in said basis and said plug at said transitions. These flared recesses will guide the needle to attain a curved shape without sharp bending when it is bent away from its linear shape. A similar guiding, flared recess may appropriately be provided at the transition between the plug and the injection needle.

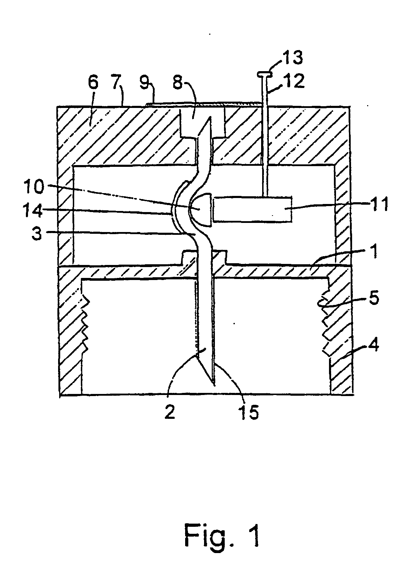

In the following the invention will be described in further details with references to the drawing, wherein
- Figure 1: shows a sectional view of a needle hub according to the invention,
- Figure 2: shows a perspective view of another embodiment of a needle hub according to the invention,
- Figure 3: shows a side view of the needle hub in figure 2,
- Figure 4: shows a sectional view along the line A-A in the needle hub in figure 3,
- Figure 5: shows the needle hub in figure 4 cocked for automatic needle insertion,
- Figure 6: shows the needle hub in figure 4 wherein the a helical spring provides some of the needle insertion force, and
- Figure 7: shows the needle hub in figure 6 cocked for automatic needle insertion.

As in a conventional needle hub the needle hub with the needle according to the invention comprises a needle, a base 1 through which the needle extends centrally forming a back needle 2 projecting from one side of the base 1 and a front needle 3 projecting from the other side of the base, the needle being fixed in the base either by a glue or by being embedded in the plastic material of the base, and a sleeve 4 depending from the perimeter of the base at the same side of this base as projects the back needle 2 which the sleeve surrounds concentrically. On its inner wall the sleeve is provided with a thread 5 by which the hub can be screwed onto a not shown injection syringe so that the back needle 2 pieces a closure of an ampoule in said syringe.

The needle is made from a super elastic alloy and can consequently be bent in rather sharp curves as shown in the drawing without collapsing or breaking and will try to return to its original straight shape when the bending forces are removed. Such alloys are mainly NiTi alloys.

Relative to a conventional needle hub the hub shown in the drawing has an attachment with a bottom plate 6 through which the front needle 3 can pass with a very small play so that this bottom acts as a guide for the front needle 3 when said front needle is inserted through the skin. The bottom plate has an abutment surface 7 which is placed in contact with the skin where the injection is intended to take place and when the sharp tip of the front needle is passed through the bottom plate this tip is guided into the skin as the small play between needle and bottom plate does not allow the needle to bend out.

A cavity 8 is provided in the abutment surface around the tip of the needle and the cavity is covered by a cover plate 9. When the attachment is a closed construction the cover plate 9 will act as a closure which ensures that the front needle can be kept in a sterile environment. The cavity 8 can be filled with a lubricant which makes the needle easy pass through the skin and/or an anaesthetic which makes the pain caused by the piercing not felt.

Alternatively the cover plate can have a frosted surface facing the cavity. As long as the cavity is filled with air the cavity will be seen as a dim spot on the cover plate 9. When so-called air shots are made to make the syringe ready for injection, which is done by setting a dose and pressing the injection button of the syringe without inserting the needle trough the skin, the spot over the cavity will remain dim as long as only air is injected in the cavity. As soon as liquid and not air is pressed out through the needle the cavity 8 will quickly fill this cavity and make the frosted surface clear to indicate that the syringe is ready for injection. The cover plate can be removable or can simply be pierced by the needle when this needle is moved out through the bottom plat 6 and into the tissue of the patient.

The insertion of the needle can be obtained by using a needle having a length making the straight needle project from the bottom plate 6 a distance corresponding to a conventional needle length, e. g. about 6 mm. A half circular curved is induced on the front needle 3 between the hub base 1 and the needle guiding bottom plate 6 so that the tip of the needle is positioned behind the surface 7 in the cavity 8 and the needle is kept in this position by releasable means here illustrated as a block 10. The block 10 is held in position by a support 11 provided with at least one pin 12 which runs through the bottom plate 6 and in a distance from the abutment surface 7 of said bottom plate 6 is provided with a pad 13. When the abutment surface 7 is pressed against the skin the pad 13 will first hit the skin and be pressed into the hub and the support 11 will be pressed away from the block 10 which will then be passed away by the needle which will due to its inherit spring force return to its straight shape. The needle will now project from the abutment surface 7 and into the skin of the patient. If the spring force of the needle is not sufficient to insert the needle this force can be supported by a stronger conventional spring here shown as a leaf spring 14 attached to the curved part of the needle.

A common problem by thin needles is that the back needle is not sufficiently rigid to penetrate the locking membrane of an ampoule without crumbling. To overcome this problem the back needle may be reinforced by a conventional steel needle 15 or by a plastic sheath which integral with the base material forms part of the embedding of the needle in the base.

The curved part of the needle can have any appropriate curved shape and is not restricted to the half circular shape described, and also the releasable mechanism allowing the needle to retain is straight shape can be provided in any appropriate way without deviating from the scope of the invention.

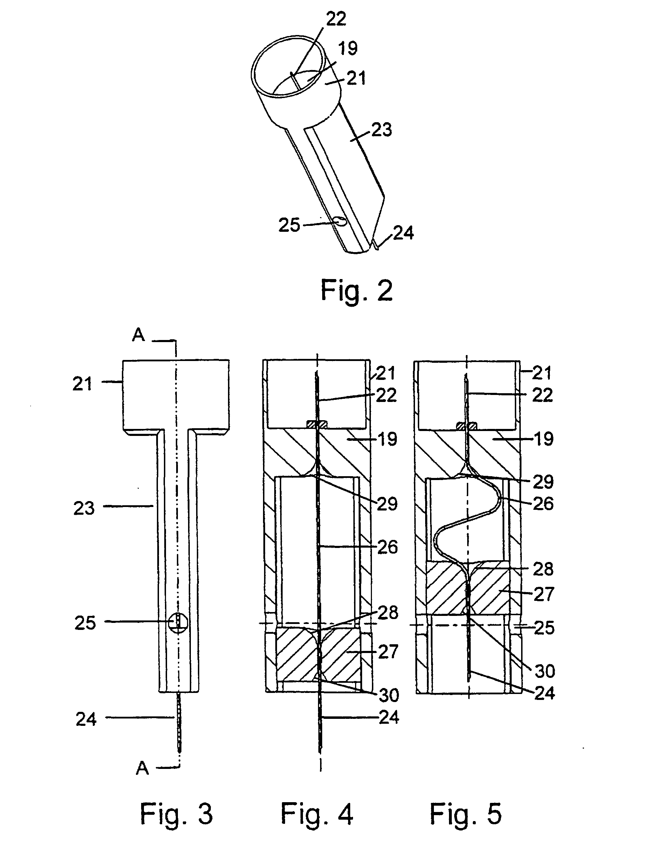

Figure 2 shows a perspective view of another embodiment of a needle with an automatic needle insertion mechanism. A sleeve 21 is provided with a not shown internal thread and can be screwed onto a socket on a syringe. By this mounting a back needle 22 penetrate a sealing of a cartridge in the syringe. The Back needle is a part of a needle which is fixed in a bottom 19 in the sleeve and runs all the way through a housing 23 to project from the opposite end of this housing as an injection needle 24.

Fig 3 shows a side view of the needle unit of figure 2 and figure 4 shows a sectional side view of the needle in figure 3 rotated 90° about its longitudinal axis. The needle 26 which connects the back needle 22 and the injection needle 24 and is integral with said back needle 22 and injection needle 24 is at its proximal end fixed in the end wall 19 which is integral with the housing 23 and at its distal end fixed in a plug 27 which is displaceable in the longitudinal direction of the housing.

As shown i figure 5, the plug 27 may be forced into the housing whereby the needle 26 is given a curved course through the housing 23. At the transitions between the needle 26 and the plug 27 and the needle 26 and the bottom 19, respectively a flared conical recess 28 and 29, respectively, are made whereby the needle 26 is guides so that it is not bend in a sharp angle at these transitions. A similar flared conical recess 30 is made around the injection needle to avoid sharp bending at the transition between the plug 27 and this injection needle 24. When the plug 27 is pressed into the housing 23 the needle is forced to the position shown in figure 5 and the elasticity of the needle 26 will seek to return to its linear appearance and will press the plug 27 in a distal direction. Movement of the plug 27 may be blocked by insertion of a not shown pin under the plug 27 through an opening 25 in the housing 23 so that the plug is held in a position in which the whole injection needle 24 is hidden in the housing 23. The distal end of the housing may now be pressed against the skin where an injection is wanted and when the not shown pin is removed from the opening 25 the needle 26 will due to its elasticity take up its linear shape and press the plug in the distal direction and thereby press the injection needle 24 through the skin ready for an injection.

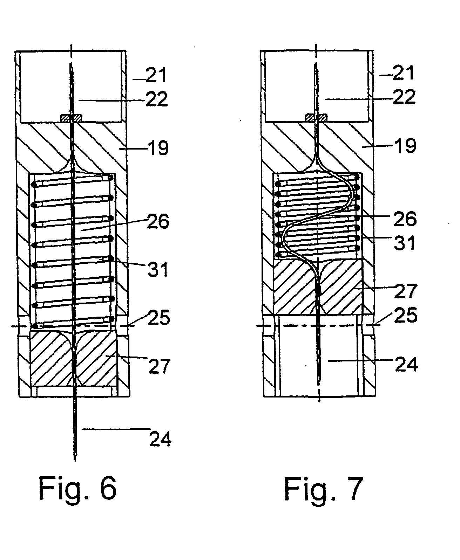

If the elasticity of the needle 26 is not sufficient to provide the force necessary to insert the injection needle 24 through the skin the force provided by the needle itself it can be supplemented by a conventional helical spring 31 as shown in figure 6 and 7 which shows sectional views corresponding to figure 4 and 5.

## Claims

1. An injection needle unit for use on a syringe by patient administrated injections, comprising a hub (1,4) fitting on the syringe which hub carries a needle (2,3) having a cross section diameter less than 0,320 mm and being mounted in the hub with pointed ends projecting from each end of the hub a first end designed for penetrating the seal of a cartridge in a syringe and a second end designed for insertion into the skin of a patient to inject medicine from said cartridge, **characterised in that** the needle is made from a super elastic material defined as a material which can obtain recovered elongation of more than 2% without having permanent deformation in the material.

2. An injection needle unit according to claim 1, **characterised in that** the needle is made from a super elastic material which belongs to a special group of materials which in a given temperature interval change their crystal structure.

3. An injection needle unit according claim 2, **characterised in that** the super elastic material used is a NiTi alloy.

4. An injection needle unit according claim 3, **characterised in that** the NiTi alloy contains at least 52 % Ni and consists of the balance Ti and unavoidable impurities.

5. An injection needle unit according claim 3 or 4, **characterised in that** the NiTi alloy contains at least one further element at a content not higher than 4%.

6. An injection needle unit according claim 1 **characterised in that** the needle is made from a curable or a thermoplastic material.

7. Injection needle unit according to anyone of the preceding claims **characterised in, that** the needle hub comprises a basis (1), a sleeve (4) with an internal thread (5), which sleeve forms attachment means and can be screwed onto an outer thread on an attachment part of a syringe, and that the needle (2,3) is fixed in the basis with an injection part projecting from one side of said basis and a back needle projecting from the other side of the basis, the back needle being surrounded by the sleeve which depends from the perimeter of the basis perpendicularly to said basis on the same side as the back needle.

8. Injection needle unit according to anyone of the preceding claims, **characterised in that** the hub has a needle guide (6) comprising an abutment surface (7) for abutment with the skin where the injection is going to be made, and a needle guide channel opening through said abutment surface through which needle guide channel the needle is movable relative to the abutment surface to make a sharpened end of the needle project from said surface.

9. Injection needle unit according to claim 8 **characterised in that** a cavity (8) is provided in the abutment surface around the opening of the needle guide channel which cavity is covered by a cover plate (9) sealed to the abutment surface.

10. Injection needle unit according to claim 9, **characterised in that** a surface of the cover plate facing the cavity is frosted.

11. Injection needle unit according to claim 9, **characterised in that** the cavity is filled with a lubricant.

12. Injection needle unit according to claim 9 or 11, **characterised in that** the cavity is filled with an anaesthetic.

13. Injection needle unit according to anyone of the claims 8 to 12, **characterised in that** means (10, 11) are provided enforcing a curved course of the needle between the basis of the needle hub and the needle guide so that the sharp point of the needle is positioned immediately beneath the abutment surface, and that release means (12, 13), are provided which removes said enforcement so that the needle due to its elasticity recovers a straight shape.

14. Injection needle unit according to claim 12 **characterised in that** a conventional spring element (31) is provided supporting the straightening of the needle.

15. Injection needle unit according to claim 14 **characterised in that** the conventional spring element is a leaf spring adjacent to the curved part of the needle.

16. Injection needle unit according to anyone of the preceding claims, **characterised in that** a reinforcement (15) of the back needle is provided.

17. Injection needle unit according to claim 16, **characterised in that** the reinforcement of the back needle comprises a plastic needle integral with the needle hub surrounding the back needle of the super elastic needle.

18. injection needle unit according to claim 16, **characterised in that** the reinforcement of the back needle is a conventional stainless steel needle (15) mounted in the needle hub.

19. An Injection needle unit according to anyone of the claims 1-6 **characterised in, that** it comprises a needle hub comprising a basis (19) having a first and a second side, a sleeve (21) projecting from the first side of the basis and forming attachment means by which it can be mounted onto an attachment part of a syringe, and a housing (23) projecting from the second side of the basis in which housing a plug (27) is axially displaceable a needle (26) fixed in the basis with a part forming a back needle (22) projecting from the first side of the basis and being surrounded by the sleeve, and rest of the needle (24) projecting from the second side of the basis and being surrounded by the housing, the needle further being fixed in the plug with an injection part projecting from a distal end of the plug and an intermediary part running between a proximal end of the plug and the second side of the basis.

20. An injection needle unit according to claim 19, **characterised in that** a guiding flared recess is provided around the needle at the transition between the plug and the injection needle.

## Patentansprüche

1. Injektionsnadeleinheit zur Verwendung an einer Spritze für durch den Patienten, verabreichten Injektionen, umfassend eine auf der Spritze angebrachte Nabe (1, 4), wobei die Nabe eine Nadel (2, 3) mit einem Querschnittdurchmesser von weniger als 0,320 mm trägt, die in der Nabe angebracht ist, indem spitz zulaufende Enden sich von jedem Ende der Nabe erstrecken, wobei ein erstes Ende zum Durchdringen der Abdichtung einer Patrone in einer Spritze und ein zweites Ende zum Einführen in die Haut eines Patienten bestimmt ist, um Medizin aus der Patrone zu injizieren, **dadurch gekennzeichnet, dass** die Nadel aus einem superelastischen Material hergestellt ist, das als Material definiert ist, das eine wiederhergestellte Verlängerung von mehr als 2% erhalten kann, ohne eine dauerhafte Verformung im Material aufzuweisen.

2. Injektionsnadeleinheit nach Anspruch 1, **dadurch gekennzeichnet, dass** die Nadel aus einem superelastischen Material hergestellt ist, das zu einer besonderen Gruppe von Materialien gehört, die in einem vorgegebenen Temperaturintervall ihre Kristallstruktur ändern.

3. Injektionsnadeleinheit nach Anspruch 2, **dadurch gekennzeichnet, dass** das verwendete superelastische Material eine NiTi-Legierung ist.

4. Injektionsnadeleinheit nach Anspruch 3, **dadurch gekennzeichnet, dass** die NiTi-Legierung mindestens 52% Ni enthält und der Rest aus Ti und unvermeidlichen Verunreinigungen besteht.

5. Injektionsnadeleinheit nach Anspruch 3 oder 4, **dadurch gekennzeichnet, dass** die NiTi-Legierung mindestens ein weiteres Element mit einem Gehalt von nicht höher als 4% enthält.

6. Injektionsnadeleinheit nach Anspruch 1, **dadurch gekennzeichnet, dass** die Nadel aus einem härtbaren oder thermoplastischen Material hergestellt ist.

7. Injektionsnadeleinheit nach einem der vorangehenden Ansprüche, **dadurch gekennzeichnet, dass** die Nadelnabe eine Basis (1), eine Hülse (4) mit einem Innengewinde (5) umfasst, wobei die Hülse ein Anschlusselement bildet und auf ein Außengewinde auf einem Anschlussteil einer Spritze geschraubt werden kann, und dass die Nadel (2, 3) auf der Basis mit einem sich von einer Seite der Basis erstreckenden Injektionsteil und einer sich von der anderen Seite der Basis erstreckenden, hinteren Nadel befestigt ist, wobei die hintere Nadel von der Hülse umgeben ist, die von dem Umfang der Basis abhängt, das senkrecht zu der Basis auf derselben Seite wie die hintere Nadel liegt.

8. Injektionsnadeleinheit nach einem der vorangehenden Ansprüche, **dadurch gekennzeichnet, dass** die Nabe eine Nadelführung (6), die eine Anliegefläche (7) zum Anliegen an der Haut, wo die Injektion durchgeführt wird, umfasst, und eine Nadelführungskanalöffnung durch die Anliegefläche aufweist, wobei durch den Nadelführungskanal die Nadel in Bezug auf die Anliegefläche entfernbar ist, um zu erreichen, dass ein angespitztes Ende der Nadel aus der Fläche herausragt.

9. Injektionsnadeleinheit nach Anspruch 8, **dadurch gekennzeichnet, dass** ein Hohlraum (8) in der Anliegefläche um die Öffnung des Nadelführungskanals bereitgestellt ist, wobei der Hohlraum von einer an der Anliegefläche abgedichteten Deckplatte (9) bedeckt ist.

10. Injektionsnadeleinheit nach Anspruch 9, **dadurch gekennzeichnet, dass** eine Oberfläche der dem Hohlraum zugewandten Deckplatte mattiert ist.

11. Injektionsnadeleinheit nach Anspruch 9, **dadurch gekennzeichnet, dass** der Hohlraum mit einem Schmiermittel gefüllt ist.

12. Injektionsnadeleinheit nach Anspruch 9 oder 11, **dadurch gekennzeichnet, dass** das der Hohlraum mit einem Anästhetikum gefüllt ist.

13. Injektionsnadeleinheit nach einem der Ansprüche 8 bis 12, **dadurch gekennzeichnet, dass** Elemente (10, 11) bereitgestellt sind, die einen gekrümmten Verlauf der Nadel zwischen der Basis der Nadelnabe und der Nadelführung erzwingen, so dass der Stechpunkt der Nadel unmittelbar unterhalb der Anliegefläche positioniert ist, und dass Freisetzungselemente (12, 13) bereitgestellt sind, die die Erzwingung aufheben, so dass die Nadel aufgrund ihrer Elastizität eine gerade Form annimmt.

14. Injektionsnadeleinheit nach Anspruch 12, **dadurch gekennzeichnet, dass** ein herkömmliches Federelement (31) bereitgestellt ist, das die Begradigung der Nadel unterstützt.

15. Injektionsnadeleinheit nach Anspruch 14, **dadurch gekennzeichnet, dass** das Federelement eine Bandfeder ist, die in der Nähe des gekrümmten Teils der Nadel liegt.

16. Injektionsnadeleinheit nach einem der vorangehenden Ansprüche, **dadurch gekennzeichnet, dass** eine Verstärkung (15) auf der Rückseite der hinteren Nadel bereitgestellt ist.

17. Injektionsnadeleinheit nach Anspruch 16, **dadurch gekennzeichnet, dass** die Verstärkung der hinteren Nadel eine in der Nadelnabe eingebaute Kunststoffnadel umfasst, die die hintere Nadel der superelastischen Nadel umgibt.

18. Injektionsnadeleinheit nach Anspruch 16, **dadurch gekennzeichnet; dass** die Verstärkung der hinteren Nadel eine herkömmliche Edelstahlnadel (15) ist, die in der Nadelnabe angebracht ist.

19. Injektionsnadeleinheit nach einem der Ansprüche 1 bis 6, **dadurch gekennzeichnet, dass** sie eine Nadelnabe umfasst, die eine Basis (19) mit einer ersten und einer zweiten Seite, eine Hülse (21), die sich von der ersten Seite der Basis erstreckt und ein Anschlusselement bildet, durch welches sie an einen Anschlussteil einer Spritze angebracht werden kann, und ein sich von der zweiten Seite der Basis erstreckendes Gehäuse (23), wobei in dem Gehäuse ein Stopfen (27) axial verschiebbar ist, eine in der Basis befestigte Nadel (26), wobei ein Teil eine hintere Nadel (22) bildet, die sich von der ersten Seite der Basis erstreckt und von einer Hülse umgeben ist, und der Rest der Nadel (24) sich von der zweiten Seite der Basis erstreckt und von dem Gehäuse umgeben ist, wobei die Nadel ferner in dem Stopfen befestigt ist, wobei sich ein Injektionsteil von einem distalen Ende des Stopfens erstreckt und ein Zwischenteil zwischen einem proximalen Ende des Stopfens und der zweiten Seite der Basis verläuft, umfasst.

20. Injektionsnadeleinheit nach Anspruch 19, **dadurch gekennzeichnet, dass** eine führungserweiterte Ausbuchtung um die Nadel am Übergangspunkt zwischen dem Stopfen und der Injektionsnadel bereitgestellt ist.

## Revendications

1. Unité d'aiguille à injection destinée à être utilisée sur une seringue pour des injections administrées au patient, comprenant un raccord (1,4) qui s'adapte sur la seringue, dont le raccord supporte une aiguille (2, 3) dotée d'un diamètre de section transversale inférieur à 0,320 mm et qui est montée dans le raccord avec les extrémités pointues qui se projettent à partir de chaque extrémité du raccord, une première extrémité conçue pour pénétrer dans le joint d'étanchéité d'une cartouche dans une seringue et une seconde extrémité conçue pour être insérée dans la peau d'un patient afin d'injecter le médicament à partir de ladite cartouche, **caractérisée en ce que** l'aiguille est réalisée à partir d'un matériau super élastique, comme un matériau qui peut obtenir un allongement recouvré supérieur à 2% sans présenter de déformation permanente dans le matériau.

2. Unité d'aiguille à injection selon la revendication 1, **caractérisée en ce que** l'aiguille est réalisée à partir d'un matériau super élastique qui appartient à un groupe spécial de matériaux qui dans un intervalle de température donné changent leur structure cristalline.

3. Unité d'aiguille à injection selon la revendication 2, **caractérisée en ce que** le matériau super élastique utilisé est un alliage de NiTi.

4. Unité d'aiguille à injection selon la revendication 3, **caractérisée en ce que** l'alliage de NiTi contient au moins 52% de Ni et se compose du complément de Ti et des impuretés inévitables.

5. Unité d'aiguille à injection selon la revendication 3 ou 4, **caractérisée en ce que** l'alliage de NiTi contient au moins un autre élément selon une teneur non supérieure à 4%.

6. Unité d'aiguille à injection selon la revendication 1, **caractérisée en ce que** l'aiguille est réalisée à partir d'un matériau polymérisable ou thermoplastique.

7. Unité d'aiguille à injection selon l'une quelconque des revendications précédentes **caractérisée en ce que** ce raccord d'aiguille comprend une base (1), un manchon (4) avec un filetage (5) interne, dont le manchon forme des moyens de fixation et peut être vissé sur un filetage externe sur une partie de fixation d'une seringue, et **en ce que** l'aiguille (2, 3) est fixée sur la base avec une partie d'injection se projetant à partir d'un côté de ladite base et une aiguille arrière se projetant à partir de l'autre côté de la base, l'aiguille arrière étant entourée par le manchon qui dépend du périmètre de la base de manière perpendiculaire à ladite base du même côté que l'aiguille arrière.

8. Unité d'aiguille à injection selon l'une quelconque des revendications précédentes, **caractérisée en ce que** le raccord possède un guide (6) d'aiguille comprenant une surface de butée (7) pour la butée avec la peau où l'injection est en train d'être pratiquée, et une ouverture de canal de guide d'aiguille à travers ladite surface de butée, et à travers ce canal de guide d'aiguille, l'aiguille est mobile par rapport à la surface de butée pour réaliser une extrémité affûtée de l'aiguille qui se projette à partir de ladite surface.

9. Unité d'aiguille à injection selon la revendication 8 **caractérisée en ce qu'**une cavité (8) est prévue dans la surface de butée autour de l'ouverture du canal de guide d'aiguille, cette cavité est couverte par une plaque de couvercle (9) appliquée hermétiquement sur la surface de butée.

10. Unité d'aiguille à injection selon la revendication 9, **caractérisée en ce qu'**une surface de la plaque de couvercle orientée en face de la cavité est gelée.

11. Unité d'aiguille à injection selon la revendication 9, **caractérisée en ce que** la cavité est remplie avec un lubrifiant.

12. Unité d'aiguille à injection selon la revendication 9 ou 11, **caractérisée en ce que** la cavité est remplie avec un anesthésiant.

13. Unité d'aiguille à injection selon l'une quelconque des revendications 8 à 12, **caractérisée en ce que** l'on prévoit des moyens (10, 11) qui imposent une course courbe de l'aiguille entre la base du raccord de l'aiguille et le guide d'aiguille de sorte que le point affûté de l'aiguille est positionné immédiatement sous la surface de butée, et **en ce que** l'on prévoit des moyens de libération (12, 13) qui retirent ladite force de sorte que l'aiguille retrouve une forme droite grâce à son élasticité.

14. Unité d'aiguille à injection selon la revendication 12 **caractérisée en ce qu'**un élément de ressort classique (31) est prévu pour supporter le redressement de l'aiguille.

15. Unité d'aiguille à injection selon la revendication 14, **caractérisée en ce que** l'élément de ressort classique est un ressort à lame adjacent à la partie courbe de l'aiguille.

16. Unité d'aiguille à injection selon l'une quelconque des revendications précédentes, **caractérisée en ce que** l'on propose un renforcement (15) de l'aiguille arrière.

17. Unité d'aiguille à injection selon la revendication 16, **caractérisée en ce que** le renforcement de l'aiguille arrière comprend une aiguille en plastique solidaire avec le raccord d'aiguille entourant l'aiguille arrière de l'aiguille super élastique.

18. Unité d'aiguille à injection selon la revendication 16, **caractérisée en ce que** le renforcement de l'aiguille arrière est une aiguille en acier inoxydable (15) classique montée dans le raccord d'aiguille.

19. Unité d'aiguille à injection selon l'une quelconque des revendications 1-6, **caractérisée en ce qu'**elle comprend un raccord d'aiguille comprenant une base (19) ayant un premier et un second côté, un manchon (21) se projetant à partir du premier côté de la base et formant des moyens de fixation avec lesquels il peut être monté sur une partie de fixation d'une seringue, et un logement (23) se projetant à partir du second côté de la base, logement dans lequel un bouchon (27) peut être déplacé de manière axiale, une aiguille (26) fixée dans la base avec une partie formant une aiguille arrière (22) se projetant à partir du premier côté de la base et étant entourée par le manchon, et le reste de l'aiguille (24) se projetant à partir du second côté de la base et étant entouré par le logement, l'aiguille étant en outre fixée dans le bouchon avec une partie d'injection se projetant à partir d'une extrémité distale du bouchon et une partie intermédiaire s'étendant entre une extrémité proximale du bouchon et le second côté de la base.

20. Unité d'aiguille à injection selon la revendication 19, **caractérisée en ce qu'**un enfoncement évasé de guidage est prévu autour de l'aiguille au niveau de la transition située entre le bouchon et l'aiguille à injection.
